# EUROPÄISCHE PATENTSCHRIFT

(11) **EP 1 734 064 B1**
(45) Veröffentlichungstag und Bekanntmachung des Hinweises auf die Patenterteilung: **06.08.2008**
(21) Anmeldenummer: 06020911.1
(22) Anmeldetag: 22.01.2004
(51) Int. Cl.: C08G 73/02, C07C 279/12, A61K 31/155, A61P 35/00

(54) **Polymere Guanidinderivate als zytostatische Arzneimittel**
Polymer guanidine derivatives as a cytostatic medicament
Dérives de guanidine polymeres utilises comme medicament cytostatique

(30) Priorität: 04.02.2003 AT 1742003
(43) Veröffentlichungstag der Anmeldung: 20.12.2006
(62) Teilanmeldung aus: 04704150.4
(73) Patentinhaber: Geopharma ProduktionsgmbH, 1210 Wien (AT)
(72) Erfinder: Schmidt, Oskar, 1030 Wien (AT)
(74) Vertreter: Schwarz, Albin

(56) Entgegenhaltungen:
- EP-A- 0 439 698
- EP-A- 0 439 699
- WO-A-01/85676
- US-A- 2 336 605
- "Cross:linked copolymers of poly:oxy:alkylene and poly:alkylene:guanidine as surfactants - prepd by heating poly:hexa:methylene:guanidine with ethylene or propylene oxides in the presence of alkali" DERWENT, 23. Mai 1993 (1993-05-23), XP002174284

## Beschreibung

Die Erfindung betrifft ein Arzneimittel, insbesondere ein Zytostatikum.

In der westlichen Zivilisation erkrankt ein Drittel der Menschen an Krebs mit einer Mortalitätsrate von nicht weniger als 75%. Bösartige Tumore werden heutzutage zytostatisch behandelt.

In der Chemotherapie dieser Erkrankungen besteht das Hauptproblem darin, dass die Krebszellen nur zu einem bestimmten Prozentsatz auf das applizierte Zytostatikum ansprechen. Darüberhinaus kann selbst bei Ansprechen des Tumors auf die Behandlung oft keine vollständige Remission erwartet werden.

Ein aktueller Trend zur Effizienzsteigerung der Chemotherapie ist die Polychemotherapie, d.i. der Einsatz mehrerer Zytostatika. Immer häufiger werden zur Verbesserung der Krebstherapie verschiedene Zytostatika mit verschiedenen Angriffspunkten kombiniert. Dadurch wird einerseits eine bessere Wirkung erzielt und andererseits dem Problem einer zunehmenden Resistenzentwicklung entgegengewirkt.

Eine weitere Möglichkeit besteht darin, durch Mitverabreichung von Zytoprotektoren die gesunden Zellen vor dem Zytostatikum selektiv zu schützen, wodurch eine höhere Dosis bei gleichzeitig geringeren Nebenwirkungen verabreicht werden kann (z.B. Taxane).

Trotz dieser Maßnahmen ist die Nebenwirkungsrate bei der Chemotherapie immer noch sehr hoch. Gerade aus diesem Grund ist es von großer Bedeutung, Wirkstoffe zu entwickeln, die sowohl eine gute Wirksamkeit als auch eine gute Verträglichkeit aufweisen, d.h. ein möglichst breites therapeutisches Fenster besitzen.

Die Erfindung setzt sich zum Ziel, ein Zytostatikum zur Verfügung zu stellen, welches im Vergleich zu herkömmlichen Zytostatika, wie 5-Fluorouracil, Cisplatin, Epirubicin und Mitomycin C ein breiteres therapeutisches Fenster aufweist.

Das erfindungsgemäße Arzneimittel enthält als Wirkstoff ein polymeres Guanidinderivat auf Basis eines Diamins, welches Oxyalkylenketten zwischen zwei Aminogruppen enthält, wobei das Guanidinderivat ein Produkt der Polykondensation eines Guanidin-Säureadditionssalzes mit einem Diamin, welches Polyalkylenketten zwischen zwei Aminogruppen enthält, darstellt, sowie dessen pharmazeutisch akzeptable Salze.

Eine bevorzugte Ausführungsform des erfindungsgemäßen Arzneimittels ist dadurch gekennzeichnet, dass als Vertreter der Reihe der Polyoxyalkylen-Guanidin-Salze solche unter Einsatz von Triethylenglykoldiamin (relative Molekularmasse: 148), von Polyoxypropylendiamin (relative Molekularmasse: 230) sowie von Polyoxyethylendiamin (relative Molekularmasse: 600) sind.

Als Wirkstoff ist besonders bevorzugt Poly-[2-(2-ethoxy-ethoxyethyl)-guanidiniumhydrochlorid] mit mindestens 3 Guanidiniumresten enthalten, wobei die mittlere Molekularmasse insbesondere im Bereich 500 und 3.000 D liegt.

Die Erfindung betrifft ferner die Verwendung eines polymeren Guanidinderivates auf Basis eines Diamins, welches Oxyalkylenketten zwischen zwei Aminogruppen enthält, wobei das Guanidinderivat ein Produkt der Polykondensation eines Guanidin-Säureadditionssalzes mit einem Diamin, welches Polyalkylenketten zwischen zwei Aminogruppen enthält, darstellt, sowie deren pharmazeutisch akzeptable Salze, zur Herstellung eines zytostatisch wirksamen Arzneimittels.

Ferner betrifft die Erfindung die Verwendung der Polyoxyalkylen-Guanidin-Salze, hergestellt unter Einsatz von Triethylenglykoldiamin (relative Molekularmasse: 148), von Polyoxypropylendiamin (relative Molekularmasse: 230) sowie von Polyoxyethylendiamin (relative Molekularmasse: 600).

Die erfindungsgemäß eingesetzten polymeren Guanidinderivate sind aus der PCT/AT01/00134 bekannt. Durch die Bezugnahme wird der Inhalt dieser Literatur in die vorliegende Beschreibung aufgenommen.

Die Herstellung eines bevorzugten Vertreters der erfindungsgemäß eingesetzten Verbindungen sowie der Nachweis der zytostatischen Wirksamkeit werden nachfolgend beschrieben.

Stellvertretend für die erfndungsgemäß eingesetzte Verbindungsklasse wird nachfolgend die zytostatische Wirksamkeit von Poly-[2-(2-ethoxy-ethoxyethyl)-guanidinium-hydrochlorid] mit einer mittleren Molekularmasse von 1000 D beschrieben (CAS Nr. 374572-91-5).

Zur Herstellung dieser Verbindung wurden 4,43 Mol Guanidiniumhydrochlorid in 4,03 Mol Triethylenglycoldiamin bei 50°C gelöst. Anschließend wurde auf 120°C erwärmt und 2 Stunden bei dieser Temperatur gerührt. Danach wurde die Temperatur 2 Stunden gehalten, dann ein Vakuum (0,1 bar) angelegt und 2 weitere Stunden unter Vakuum bei 170°C gerührt. Anschließend wurde auf Normaldruck belüftet, auf 120°C abkühlen gelassen und mit entmineralisiertem Wasser auf ca. 50% verdünnt. Mit Phosphorsäure wurde auf einen pH von ca. 6 neutralisiert, abkühlen gelassen und auf die gewünschte Konzentration verdünnt. Das Molekulargewicht wurde mit 1000 D bestimmt.

### Nachweis der zytostatischen Wirksamkeit

Untersucht wurden Zellreihen von Colon-Karzinom und Pancreas-Karzinom, wie Capan-1, DLD-1, HT 29, HCT-8, MIA-PA-CA2, PANC1, BXPC-3, ASPC-1, und HT-29. Die getesteten Karzinomzellreihen wurden in flüssigem Stickstoff aufbewahrt. Nach dem Auftauen wurden die Karzinomzellen in Kulturflaschen mit RPMI-1640 + Glutamin Medium (Gibco Nr. 5240025) bei 37°C/5 % CO₂ Atmosphäre bis zu 14 Tagen kultiviert, so daß sich ein Monolayer von Zellen bilden konnte. Danach wurden die Zellen mit Trypsin + EDTA (Gibco Nr. 15400-054) geerntet und mit RPMI-Medium zweimal gewaschen.

Darüber hinaus wurden Lymphozyten von gesunden Probanden untersucht. Hierbei wurde eine Gesamtmenge von 100 ml Blut in EDTA-Röhrchen entnommen. Die Lymphozyten wurden aus dem Vollblut mittels Mono-Poly-Resolving Medium/Ficol-Hypaque-gradient gewonnen, dreimal mit HBSS-Puffer gewaschen und auf diese Weise vorbereitet in den Testansatz eingesetzt.

Neben dem Poly-[2-(2-ethoxy-ethoxyethyl)-guanidinium-hydrochlorid] wurden die Chemotherapeutika Cisplatin, Epirubicin, Mitomycin-C und 5-Fluorouracil als Vergleich getestet. Dazu wurden die Verbindungen zunächst nach den jeweiligen Vorschriften des Herstellers gelöst und als Stammlösung zu je 1 ml (Wirkstoffkonzentration 1000 µg/ml) bei - 180°C in flüssigem Stickstoff aufbewahrt. Gelöste Substanzen wurden noch am selben Tag verwendet.

Für die Bestimmung der zytotoxischen Aktivität der Substanzen wurden die Karzinomzellen aus den Kulturflaschen mit RPMI gewaschener Suspension in einer Konzentration von 20.000 Zellen in 200 µl in Mikrotiter-Platten überführt. In Gegenwart von variierenden Konzentrationen der Testsubstanzen wurden die Karzinomzellen anschließend bei einer Temperatur von 37°C und 5 % CO₂-Atmosphäre für drei Tage inkubiert. Für das Poly-[2-(2-ethoxy-ethoxyethyl)-guanidinium-hydrochlorid] wurden Konzentrationen von 0,12 bis 500 µg/ml (Faktor 2 Verdünnungen) in allen Versuchsanordnungen verwendet. Nach der dreitägigen Bebrütungszeit erfolgte die Evaluierung mittels des nichtradioaktiven Zellproliferations- und Zytotoxizitätstests EZ4U (Biomedica Nr. BI-5000 10 x 96 Bestimmungen). Nach drei Stunden Inkubation mit EZ4U erfolgte photometrisch die Evaluierung bei einer Wellenlänge von 49/630 nm im Dias-Photometer (Extinktion der Testprobe durch Extinktion des Kontrollprobe-Leerwertes) in Prozent.

Die Vitalität der Lymphozyten wurde in einer Suspension von 1,2 x 10⁷/ml in variierenden Konzentrationen der Prüfsubstanzen bestimmt. Die Zellsuspensionen wurden in Gegenwart der Prüfsubstanzen für 24 Stunden bei 37°C und 5 %iger CO₂ Atmosphäre inkubiert und danach mit Trypan-Blau gefärbt. Nach der Färbung wurden die Lymphozyten auf die Zählkammer aufgebracht und auf ihre Vitalität in Prozent evaluiert.

Der Wirkstoff Poly-[2-(2-ethoxy-ethoxyethyl)-guanidinium-hydrochlorid] besitzt, bei geringer Toxizität und guter Verträglichkeit aus pharmakologischer Sicht, günstige pharmakodynamische Eigenschaften und kann daher als Heilmittel in der onkologischen Therapie verwendet werden. Insbesondere zeigt die Substanz eine ausgezeichnete zytostatische Wirksamkeit, wie sich durch Untersuchungen unter Verwendung von mehreren Karzinomzellreihen, wie z.B. Colonkarzinom (HT-29, HCT-8, DLD-1) oder Pankreaskarzinom (ASPC-1, BXPC-3, CAPAN-1, PANC-1) zeigen läßt.

Zudem besitzt der erfindungsgemäß eingesetzte Wirkstoff ein breites therapeutisches Fenster, denn während die zytostatische Wirkung bei Karzinomzellen bereits ab einer Konzentration 2 bis 16 µg/ml auftritt (Tabelle 1 und 2), wurde bei gesunden, körpereigenen Zellen, wie Lymphozyten, diese Wirkung erst bei Konzentrationen ab 100 µg/ml beobachtet (Tabelle 4). Tabelle 3 zeigt zum Vergleich die zytostatische Wirksamkeit von 5-Fluorouracil, Cisplatin, Epirubicin und Mitomycin C.

Nach systemischer Verabreichung (intravenös oder intraperitoneal) von Poly-[2-(2-ethoxy-ethoxyethyl)-guanidinium-hydrochlorid]in Mengen bis zu 15 mg/kg Körpergewicht werden in der Ratte im Blut Serumkonzentrationen bis zu 100 µg/ml nach zwei Stunden gemessen, bei gleichzeitig guter Verträglichkeit. Daher kann Poly-[2-(2-ethoxy-ethoxyethyl)-guanidinium-hydrochlorid] als Zytostatikum verwendet werden.

Die erfindurigsgemäß eingesetzten Wirkstoffe können in an sich bekannter Weise zu pharmazeutischen Präparaten vearbeitet werden, und zwar allein oder gemeinsam mit anorganischen oder organischen, pharmakologisch indifferenten Hilfsstoffen.

**Tabelle 1. Zytostatische Aktivität gegenüber verschiedenen Colonkarzinom-Zellreihen (Vitalität in %)**

| Testverbindung* | | | | | | |
|---|---|---|---|---|---|---|
| **Konzentration (µg/ml)** | **HAT-29** | **HCT-8** | **DLD-1** | **HCT-15** | **Colon 320DM** | **Colon 205** |
| 64 | 11 | 11 | 15 | 16 | 21 | 15 |
| 32 | 13 | 15 | 17 | 18 | 22 | 16 |
| 16 | 21 | 34 | 26 | 25 | 24 | 16 |
| 8 | 47 | 91 | 49 | 69 | 26 | 27 |
| 4 | 76 | 114 | 86 | 81 | 37 | 59 |
| 2 | 77 | 90 | 103 | 81 | 79 | 70 |
| 8 | 88 | 85 | - | - | - | |
| 0,5 | 98 | 79 | - | - | - | |
| 0,25 | 85 | 70 | - | - | - | |
| 0,125 | 76 | 88 | - | - | - | |

| | | | | | | |
|---|---|---|---|---|---|---|
| * Poly-[2-(2-ethoxy-ethoxyethyl)-guanidiniumhydrochlorid] | | | | | | |

**Tabelle 2. Zytostatische Aktivität gegenüber verschiedenen Pankreaskarzinom-Zellreihen (Vitalität in %)**

| Testverbindung* | | | | | |
|---|---|---|---|---|---|
| **Konzentration (µg/ml)** | **AsPc-1** | **BxPc-3** | **Capan-1** | **Panc-1** | **MIA PaCa-2** |
| 64 | 13 | 9 | 46 | 11 | 11 |
| 32 | 12 | 11 | 79 | 16 | 12 |
| 16 | 15 | 18 | 114 | 30 | 16 |
| 8 | 50 | 44 | 95 | 55 | 58 |
| 4 | 70 | 74 | 111 | 90 | 97 |
| 2 | 92 | 63 | 115 | 88 | 106 |

| | | | | | |
|---|---|---|---|---|---|
| * Poly-[2-(2-ethoxy-ethoxyethyl)-guanidiniumhydrochlorid] | | | | | |

**Tabelle 3. Zytostatische Aktivität von Standardchemotherapeutika gegenüber verschiedenen Pankreaskarzinom-Zellreihen (Vitalität in %)**

| **Substanzkonzentration in µg/ml** | | **AaPc-1** | **BxPc-3** | **Capan-1** | **Panc-1** |
|---|---|---|---|---|---|
| **5-Fluorouracil** | 60 | 71 | 67 | 23 | 62 |
| | 6 | 88 | 79 | 38 | 77 |
| | 0,6 | 92 | 86 | 46 | 80 |
| **Cisplatin** | 2,5 | 53 | 93 | 36 | - |
| | 0,25 | 89 | 65 | 54 | - |
| | 0,025 | 85 | 87 | 94 | - |
| **Epirubicin** | 11,9 | 55 | 62 | 54 | 61 |
| | 1,19 | 59 | 55 | 35 | 43 |
| | 0,12 | 105 | 76 | 50 | 111 |
| **Mitomycin C** | 0,5 | 65 | 47 | 22 | 55 |
| | 0,15 | 89 | 91 | 30 | 103 |
| | 0,015 | 98 | 100 | 59 | 104 |

**Tabelle 4. Vitalität von Lymphozyten in %**

| Testverbindung* **Konzentration (µg/ml)** | |
|---|---|
| 1000 | 1 |
| 500 | 16 |
| 250 | 30 |
| 100 | 98 |
| 50 | 96 |
| 25 | 100 |
| 12,5 | 98 |

| | |
|---|---|
| * Poly-[2-(2-ethoxy-ethoxyethyl)-guanidiniumhydrochlorid] | |

## Patentansprüche

1. Polymeres Guanidinderivat auf Basis eines Diamins, welches Oxyalkylenketten zwischen zwei Aminogruppen enthält, wobei das Guanidinderivat ein Produkt der Polykondensation eines Guanidin-Säureadditionssalzes mit einem Diamin, welches Polyoxyalkylenketten zwischen zwei Aminogruppen enthält, darstellt, sowie dessen pharmazeutisch akzeptable Salze, zur Verwendung als Arzneimittel.

2. Polymeres Cruanidinderivat nach Anspruch 1, **dadurch gekennzeichnet, dass** als Vertreter der Reihe der Polyoxyalkylen-Guanidin-Salze solche unter Einsatz von Triethylenglykaldiamin (relative Molekularmasse: 148), von Polyoxypropylendiamin (relative Molekularmasse: 230) sowie von Polyoxyethylendiamin (relative Molekularmasse: 600) sind.

3. Polymeres Guanidinderivat nach einem der Ansprüche 1 oder 2, **dadurch gekennzeichnet, dass** als Wirkstoff Poly-[2-(2-ethoxy-ethoxyethyl)-guanidinium-hydrochlorid] mit mindestens 3 Guanidiniumresten enthalten ist.

4. Polymeres Guanidinderivat nach Anspruch 3, **dadurch gekennzeichnet, dass** die mittlere Molekularmasse des Wirkstoffes im Bereich 500 bis 3.000 liegt.

## Claims

1. A polymeric guanidine derivative based on a diamine containing oxyalkylene chains between two amino groups, with the guanidine derivative representing a product of polycondensation between a guanidine acid addition salt and a diamine containing polyoxyalkylene chains between two amino groups, as well as the pharmaceutically acceptable salts thereof, for use as a drug composition.

2. A polymeric guanidine derivative according to claim 1, **characterized in that,** among the representatives of the family of polyoxyalkylene guanidine salts, there are such using triethylene glycol diamine (relative molecular mass: 148), polyoxypropylene diamine (relative molecular mass: 230) as well as polyoxyethylene diamine (relative molecular mass: 600).

3. A polymeric guanidine derivative according to any of claims 1 or 2, **characterized in that** poly-[2-(2-ethoxyethoxyethyl)guanidinium hydrochloride] comprising at least 3 guanidinium groups is contained as the drug substance.

4. A polymeric guanidine derivative according to claim 3, **characterized in that** the average molecular mass of the drug substance ranges from 500 to 3.000.

## Revendications

1. Dérivé polymère de guanidine à base d'une diamine qui contient des chaînes d'oxyalkylène entre deux groupes amino, le dérivé du guanidine étant un produit de polycondensation d'un sel d'addition d'acide guanidinique avec une diamine qui contient une chaîne de polyoxyalkylène entre deux groupes amino, ainsi que ses sels pharmaceutiquement acceptables, en vue d'une utilisation comme médicament.

2. Dérivé polymère de guanidine selon la revendication 1, **caractérisé en ce que** les représentants de la série des sels de polyoxyalkylène-guanidine sont ceux obtenus en recourant à une triéthylèneglycoldiamine (poids moléculaire relatif : 148), une polyoxypropylènediamine (poids moléculaire moyen : 230) ou à une polyoxyéthylènediamine (poids moléculaire relatif: 600).

3. Dérivé polymère de guanidine selon l'une des revendications 1 ou 2, **caractérisé en ce qu'**il contient comme substance active le poly-[chlorhydrate de 2-(2-éthoxy-éthoxyéthyle)-guanidinium] avec au moins 3 groupes guanidinium.

4. Dérivé polymère de guanidine selon la revendication 3, **caractérisé en ce que** le poids moléculaire moyen de la substance active est compris dans la plage de 500 à 3 000.
